# EUROPÄISCHE PATENTANMELDUNG

(11) **EP 0 748 788 A1**
(43) Veröffentlichungstag der Anmeldung: **18.12.1996**
(21) Anmeldenummer: 96108857.2
(22) Anmeldetag: 03.06.1996
(51) Int. Cl.: C07C 205/06, C07C 201/08

(54) **Verfahren zur Herstellung von Dinitrotoluol**

(30) Priorität: 14.06.1995 DE 19521614
(71) Anmelder: BAYER AG, 51368 Leverkusen (DE)
(72) Erfinder: Klingler, Uwe, 41539 Dormagen (DE); Schieb, Thomas, Dr., 51503 Rösrath (DE); Wiechers, Gerhard, Dr., 51381 Leverkusen (DE)

(57) **Zusammenfassung**

Die vorliegende Erfindung betrifft ein einstufiges Verfahren zur kontinuierlichen Herstellung von Dinitrotoluolisomerengemischen durch Nitrierung von Mononitrotoluolisomerengemischen, dadurch gekennzeichnet, daß man Mononitrotoluol und Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen, und (ii) 0 bis 20 Gew.-% organischen Bestandteilen, die zu 70 bis 100 % aus Nitrotoluol-Isomeren und zum Rest aus Nebenprodukten bestehen, unter adiabatischen Bedingungen zur Reaktion bringt, wobei das Molverhältnis von Salpetersäure zu Mononitrotoluol 0,7:1 bis 1,4:1 beträgt, anschließend die Phasen trennt und die Säurephase durch Entspannungsverdampfung gegebenenfalls unter gleichzeitiger Wärmezufuhr von mindestens 5 % des Wassers destillativ befreit und nach Zugabe von 50 bis 100 gew.-%iger Salpetersäure kontinuierlich in die Reaktion zurückführt.

## Beschreibung

Die vorliegende Erfindung betrifft ein Verfahren zur Herstellung von Dinitrotoluol durch einstufige Nitrierung von Mononitrotoluol unter adiabatischen Bedingungen.

Dinitrotoluol (DNT) ist ein Vorprodukt zur Herstellung von Toluylendiisocyanat (TDI). Technisch wird DNT durch Umsetzung von Toluol mit Nitriersäure, einem Gemisch aus Salpeter- und Schwefelsäure, gewonnen (DE-B 1 468 362, T. Urbanski, Chemistry and Technology or Explosives, Pergamon Press 1964; Ullmanns Encyclopädie der technischen Chemie, 4. Auflage, Bd. 17, S. 392, Verlag Chemie, Weinheim 1979).

Dieses Nitrierverfahren wird isotherm durchgeführt, d.h. die Reaktionswärme wird am Ort der Entstehung durch ein Kühlmittel abgeführt. Dies ist mit hohem Energieeinsatz verbunden und demzufolge aufwendig und kostspielig.

In letzter Zeit setzen sich mehr und mehr adiabatische Nitrierverfahren durch (EP 0 373 966, EP 0 436 443, US 5 313 009, EP 0 597 361). Bei diesen wird die Reaktionswärme nicht durch Kühlung abgeführt, sondern im System belassen und zum Aufkonzentrieren der Absäure genutzt. Ein weiterer Vorteil dieser adiabatischen Verfahren besteht darin, daß verdünnte Salpetersäuren verwendet werden können, die wesentlich preisgünstiger sind als die hochkonzentrierte Säure. Verdünnte Salpetersäurequalitäten können prinzipiell zwar auch bei den isothermen Verfahren verwendet werden, jedoch ist der zusätzliche Energieaufwand erheblich.

EP 0 373 966, EP 0 436 443 und US 0 597 361 beschreiben die adiabatische Nitrierung von Aromaten mit Nitriersäure, einem Gemisch aus Schwefel- und Salpetersäure. Beschrieben wird ausschließlich die Bildung von mononitrierten Verbindungen aus Kohlenwasserstoffen. Die Nitrierung eines bereits Nitrogruppen tragenden Substrates mit dem Ziel einer mehrfach nitrierten Verbindung wird nicht beschrieben. Die Ausführungsbeispiele beziehen sich ausschließlich auf die Nitrierung von Benzol. Toluol wird in keinem einzigen konkreten Beispiel als zu nitrierende aromatische Verbindung aufgeführt.

EP 0 597 361 beschreibt die Herstellung von DNT auf adiabatischem Wege. DNT wird hier einstufig durch Umsetzung von Toluol mit Nitriersäure erhalten. Auch hier wird die Mononitrierung von Mononitrotoluol zum Zwecke der DNT-Bildung nicht beansprucht.

Nachteilig bei dem in EP 0 597 361 genannten Verfahren ist der erhöhte Anteil an ortho-DNT im Vergleich zur isothermen Fahrweise. Dies ist auf die höhere Reaktionstemperatur während der Nitrierung, speziell während der Mononitrierung, zurückzuführen. Ortho-DNT ist ein unerwünschtes Gemisch aus DNT-Isomeren mit ortho-ständigen Nitrogruppen (2,3-, 3,4-DNT), welches bei der TDI-Herstellung nicht verwendet werden kann. Es stellt daher ein Abfallprodukt dar und muß mit beträchtlichem Aufwand abgetrennt und entsorgt werden.

Auch das klassiche, isotherme Nitrierverfahren liefert einen gewissen Anteil an ortho-DNT, allerdings liegt dieser nicht so hoch wie beim adiabatischen Verfahren. Die Abtrennung der ortho-DNT-Isomeren erfolgt normalerweise nach der Hydrierung auf der Amin-Stufe und erfordert eine sehr gut trennende Kolonne, da sich der Siedepunkt des abzutrennenden Produktes nur wenig von dem des gewünschten Amins unterscheidet. Aus diesem Grunde ist das Einstellen eines hohen Rücklaufverhältnisses notwendig, was die Destillationskosten deutlich in die Höhe treibt. Da für das abgetrennte ortho-Amin keine Verwendung existiert, ist ein Mehranfall an diesem Produkt einem Ausbeuteverlust gleichzusetzen. Darüber hinaus verursacht ein Mehranfall an ortho-DNT auch Zusatzkosten in der Hydrierung sowie bei der Vernichtung des unerwünschten Produktes.

Ein weiterer Nachteil dieses adiabatischen Nitrierverfahrens offenbart sich bei der Aufkonzentrierung der Absäure. Diese Absäure enthält gelöste Organika, im wesentlichen gelöstes DNT. Dieses ist wasserdampfflüchtig und wird bei der Aufkonzentrierung der Absäure weitgehend mit ausgedampft. Die modernen und sicheren Vakuumverfahren erfordern niedrige Kondensationsbedingungen für das verdampfte Wasser. Bei diesen Temperaturen kristallisiert DNT aus und führt zu Belägen im Kondensationssystem.

Dieses Problem besteht zwar auch bei isothermen Verfahren, hier behilft man sich jedoch durch Einspritzen von Mononitrotoluol (MNT) in die heißen Brüdengase (DE-A 3 409 719). Auf diese Weise wird das Brüdenkondensat durch Schmelzpunkterniedrigung flüssig gehalten und Verstopfungen werden vermieden. Dieses ist beim bekannten adiabatischen Verfahren nicht möglich, da im Verfahren kein isoliertes MNT vorliegt.

Aufgabe war es, die Herstellung von DNT auf adiabatischem Wege dahingehend zu verbessern, daß ein niedriger Gehalt an ortho-DNT resultiert und darüber hinaus die obigen Probleme bei der Aufkonzentrierung der Absäure vermieden werden.

Diese Aufgabe konnte durch das erfindungsgemäße Verfahren gelöst werden.

Gegenstand der Erfindung ist ein einstufiges Verfahren zur kontinuierlichen Herstellung von Dinitrotoluolisomerengemischen mit geringem ortho-DNT-Anteil durch Nitrierung von MNT mit einem m-Isomerengehalt kleiner gleich 4,5 Gew.-%, bevorzugt 3,5 bis 4,5 Gew.-%, welches dadurch gekennzeichnet ist, daß man MNT und Nitriersäure, bestehend aus (i) 80 bis 100 Gew.-%, bevorzugt 90 bis 100 Gew.-% an anorganischen Bestandteilen, die sich im wesentlichen aus 60 bis 90 Gew.-%, bevorzugt 65 bis 85 Gew.-% Schwefelsäure, 1 bis 20 Gew.-%, bevorzugt 1 bis 10 Gew.-% Salpetersäure und mindestens 5 Gew.-%, bevorzugt 10 bis 30 Gew.-% Wasser zusammensetzen und (ii) 0 bis 20 Gew.-%, bevorzugt 0 bis 10 Gew.-% organischen Bestandteilen, die zu 70 bis 100 %, bevorzugt 90 bis 100 % aus Nitrotoluol-Isomeren und zum Rest aus Nebenprodukten bestehen, unter adiabatischen Bedingungen zur Reaktion bringt, wobei das Molverhältnis von Salpetersäure zu MNT 0,7:1 bis 1,4:1, bevorzugt 0,8:1 bis 1,2:1 beträgt, anschließend die Phasen trennt und die Säurephase durch Entspannungsverdampfung gegebenenfalls unter gleichzeitiger Wärmezufuhr von mindestens 5, bevorzugt 5 bis 30 % des Wassers destillativ befreit und nach Zugabe von 50 bis 100, bevorzugt 60 bis 80 gew.-%iger Salpetersäure kontinuierlich in die Reaktion zurückführt.

Bevorzugt wird den Brüden aus der Aufkonzentrierung der Säurephase MNT vor deren Kondensation zugesetzt.

Die Menge an zugesetztem MNT wird so gewählt, daß das Brüdenkondensat flüssig abläuft und keine festen Beläge bildet Dies ist der Fall, wenn in der organischen Phase des Brüdenkondensates ein Gewichtsverhältnis von MNT zu DNT von 2:1 bis 10:1, bevorzugt 2:1 bis 5:1, vorliegt. Die organischen Bestandteile des Brüdenkondensates werden nach der Phasentrennung in die Nitrierstufe zurückgeführt.

Das erfindungsgemäße Verfahren wird bevorzugt bei Temperaturen von 60 bis 200°C und Drücken von 1 bis 100 bar durchgeführt.

Durch dieses erfindungsgemäße Verfahren werden ortho-DNT-Gehalte erzielt, die nicht über den Gehalten beim herkömmlichen isothermen Verfahren liegen. Durch die adiabatische Fahrweise wird die Reaktionswärme im Prozeß genutzt und es kann verdünnte Salpetersäure verwendet werden. Der Gehalt an o-DNT liegt bei dem erfindungsgemäßen Verfahren bei etwa 4,0 %,bezogen auf DNT.

Besonders vorteilhaft ist dieses Verfahren, wenn MNT, vorzugsweise auf isothermem Wege mit geringen m-Isomerenanteil hergestellt, zur Verfügung steht.

### Beispiele

### Beispiel 1

137 g/h Mononitrotoluolisomerengemisch (1 Mol/h) (Isomerenverteilung: o/m/p = 59,9/4,3/38,8 Gew.-%) und 1890 g/h (1,08 Mol/h) Nitriersäure der Zusammensetzung 77,9:3,6:18,5 (Gew.-% H₂SO₄:HNO₃:H₂O) werden bei einer Starttemperatur von ca. 120°C unter adiabatischen Bedingungen kontinuierlich zur Reaktion gebracht. Nach Trennung der Phasen wird die Säurephase im Vakuum aufkonzentriert. Um Ablagerungen im Kondensationsteil zu vermeiden, werden 9 g/h MNT in die überhitzten Brüden des Eindampfers gegeben. Die aufkonzentrierte Absäure wird nach Aufstockung mit 60 %iger Salpetersäure in den adiabatischen Nitrierschritt zurückgeführt, ebenso die organischen Bestandteile des Brüdenkondensates. Es werden 180 g/h (99 Gew.-%) Dinitrotoluolisomerengemisch isoliert. Gehalt an o-DNT: 4,1 Gew.-%.

### Beispiel 2

137 g/h (1 Mol/h) Mononitrotoluolisomerengemisch (Isomerenverteilung: o/m/p = 59,9/4,3/38,8 Gew.-%) und 587 g/h (1,08 Mol/h) Nitriersäure der Zusammensetzung 73,6:11,6:14,8 (Gew.-% H₂SO₄:HNO₃:H₂O) werden bei einer Starttemperatur von ca. 60°C unter adiabatischen Bedingungen kontinuierlich zur Reaktion gebracht. Nach Trennung der Phasen wird die Säurephase im Vakuum aufkonzentriert. Um Ablagerungen im Kondensationsteil zu vermeiden, werden 4,5 g/h MNT in die überhitzten Brüden des Eindampfers gegeben. Die aufkonzentrierte Absäure wird nach Aufstockung mit 98,5 %iger Salpetersäure in den adiabatischen Nitrierschritt zurückgeführt, ebenso die organischen Bestandteile des Brüdenkondensates. Es werden 180 g/h (99 Gew.-%) Dinitrotoluolisomerengemisch isoliert. Gehalt an o-DNT: 4,1 Gew.-%.

### Beispiel 3

137 g/h (1 Mol/h) Mononitrotoluolisomerengemisch (Isomerenverteilung: o/m/p = 59,9/4,3/38,8 Gew.-%) und 1173 g/h (1,08 Mol/h) einer Nitriersäure der Zusammensetzung 76,9:5,8:17,3 (Gew.-% H₂SO₄:HNO₃:H₂O) werden bei einer Starttemperatur von ca. 100°C unter adiabatischen Bedingungen zur Reaktion gebracht. Nach Trennung der Phasen wird die Säurephase im Vakuum aufkonzentriert. Um Ablagerungen im Kondensationsteil zu vermeiden, werden 7 g/h MNT in die überhitzten Brüden des Eindampfers gegeben. Die aufkonzentrierte Absäure wird nach Aufstockung mit 68 %iger Salpetersäure in den adiabatischen Nitrierschritt zurückgeführt, ebenso die organischen Bestandteile des Brüdenkondensates. Es werden 180 g (99 Gew.-%) Dinitrotoluolisomerengemisch isoliert. Gehalt an o-DNT: 4,0 Gew.-%.

## Patentansprüche

1. Einstufiges Verfahren zur kontinuierlichen Herstellung von Dinitrotoluolisomerengemischen durch Nitrierung von Mononitrotoluolisomerengemischen, dadurch gekennzeichnet, daß man Mononitrotoluol und Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen, und (ii) 0 bis 20 Gew.-% organischen Bestandteilen, die zu 70 bis 100 % aus Nitrotoluol-Isomeren und zum Rest aus Nebenprodukten bestehen, unter adiabatischen Bedingungen zur Reaktion bringt, wobei das Molverhältnis von Salpetersäure zu Mononitrotoluol 0,7:1 bis 1,4:1 beträgt, anschließend die Phasen trennt und die Säurephase durch Entspannungsverdampfung gegebenenfalls unter gleichzeitiger Wärmezufuhr von mindestens 5 % des Wassers destillativ befreit und nach Zugabe von 50 bis 100 gew.-%iger Salpetersäure kontinuierlich in die Reaktion zurückführt.
